# EUROPÄISCHE PATENTSCHRIFT

(11) **EP 0 519 512 B1**
(45) Veröffentlichungstag und Bekanntmachung des Hinweises auf die Patenterteilung: **21.02.1996**
(21) Anmeldenummer: 92110425.3
(22) Anmeldetag: 19.06.1992
(51) Int. Cl.: C12P 17/12

(54) **Mikrobiologisches Verfahren zur Herstellung von 5-Hydroxypyrazincarbonsäure**
Microbiological process for the preparation of 5-hydroxy pyrazinc carboxylic acid
Procédé microbiologique pour la préparation de l'acide 5-hydroxy pyrazinccarboxylique

(30) Priorität: 21.06.1991 CH 1843/91
(43) Veröffentlichungstag der Anmeldung: 23.12.1992
(73) Patentinhaber: LONZA AG, CH-3945 Gampel/Wallis (CH)
(72) Erfinder: Kiener, Andreas, Dr., Visp (Kanton Wallis) (CH)
(74) Vertreter: Weinhold, Peter, Dr.

(56) Entgegenhaltungen:
- EP-A- 0 152 948
- EP-A- 0 556 465
- EP-A- 0 558 022
- BIOCHEMICAL PHARMACOLOGY Bd. 36, Nr. 19 , 1987 Seiten 3317 - 3318 TETSUYA YAMAMOTO ET AL. 'In vitro conversion of pyrazinamide into 5-hydroxypyrazinamide and that of pyrazinoic acid into 5-hydroxypyrazinoic acid by xanthine oxidase from human liver'

## Beschreibung

Die Erfindung betrifft ein neues Verfahren zur Herstellung von 5-Hydroxypyrazincarbonsäure und/oder deren Salze mit Nikotinsäure und/oder deren Salze verwertenden Mikroorganismen ausgehend von Pyrazincarbonsäure und/oder deren Salze.

Im folgenden werden unter Nikotinsäure, Pyrazincarbonsäure und 5-Hydroxypyrazincarbonsäure auch deren Salze wie beispielsweise deren Alkalisalze oder Ammoniumsalze verstanden.

5-Hydroxypyrazincarbonsäure kann beispielsweise als Zwischenprodukt zur Herstellung von Pharmazeutika, wie beispielsweise zur Herstellung von Pyrazin-Nukleosidanalogen mit cytostatischer Wirkung (M. Bobek, J. Heterocyclic Chem., 1991, 28, S. 1131), verwendet werden.

Bekannt ist, dass 5-Hydroxypyrazincarbonsäure im Metabolismus von Hunden und Menschen aus Pyrazinamid über Pyrazincarbonsäure gebildet wird (J.Pharmacol. Exp. Ther., 1972, 180(2), 411-434).

Ein 5-stufiges chemisches Verfahren zur Herstellung von 5-Hydroxypyrazincarbonsäure wird beispielsweise ausgehend von Furfurylglyoxal (J.Heterocyclic Chem. 19, 1982, S.402) beschrieben. Dieses hat jedoch den Nachteil, dass es grosstechnisch nicht gangbar ist.

EP-A-0 152 948 offenbart ein mikrobiologisches Verfahren zur Herstellung von 6-Hydroxynikotinsäure unter Verwendung von erfindungsgemäßen Mikroorganismen.

Ein mikrobiologisches Verfahren zur Herstellung von 5-Hydroxypyrazincarbonsäure ist bis jetzt noch nicht bekannt.

Aufgabe der vorliegenden Erfindung war es, ein einfaches ökonomisches und ökologisches Verfahren zur Herstellung von 5-Hydroxypyrazincarbonsäure zur Verfügung zu stellen.

Diese Aufgabe wurde mit einem neuen Verfahren gemäss Patentanspruch 1 gelöst.

Erfindungsgemäss wird vorgeschlagen, Pyrazincarbonsäure als Substrat mit Mikroorganismen, die mit Nikotinsäure als einzige Kohlenstoff-, Stickstoff- und Energiequelle wachsen, in 5-Hydroxypyrazincarbonsäure zu überführen, wobei letztere im Medium akkumuliert wird.

Prinzipiell sind für das Verfahren alle Mikroorganismen geeignet, die Nikotinsäure über 6-Hydroxynikotinsäure abbauen. Diese Mikroorganismen können mit Hilfe üblicher mikrobiologischer Techniken beispielsweise aus Kläranlagen mit Nikotinsäure als Wachstumssubstrat isoliert werden. Beispielsweise können solche der Gattung Pseudomonas, Achromobacter, Bacillus, Azorhizobium, Sarcina und Mycobacterium angewendet werden, die bereits in der EP-A 152 948 beschrieben sind. Die Umsetzung kann sowohl mit Mischungen als auch mit Rein-Isolaten dieser Mikroorganismen, steril oder unsteril, durchgeführt werden.

Zweckmässig wird das Verfahren mit den Mikroorganismen der Spezies
Pseudomonas acidovorans DSM 4746
Achromobacter xylosoxydans DSM 2402
Achromobacter xylosoxydans DSM 2783
Pseudomonas putida NCIB 10521
Pseudomonas putida NCIB 8176
sowie deren Deszendenten und Mutanten durchgeführt, vorzugsweise mit Pseudomonas acidovorans DSM 4746.

Die Mikroorganismen der Spezies Pseudomonas acidovorans DSM 4746 wurden am 25. Juli 1988 bei der Deutsche Sammlung von Mikroorganismen und Zellkulturen GmbH, Mascheroderweg 1b, D-3300 Braunschweig, hinterlegt.

Die Mikroorganismen der Spezies Achromobacter xylosoxydans DSM 2402 und DSM 2783 sind ebenfalls an dem oben genannten Institut hinterlegt und sind bereits in der EP-A 152 948 beschrieben.

Die Mikroorganismen der Spezies Pseudomonas putida NCIB 10521 und NCIB 8176 sind bei der National Collection of Industrial Bacteria, Torry Research Station, 135 Abbey Road, Aberdeen AB98DC, Scotland, hinterlegt.

Üblicherweise wird vor der eigentlichen Umsetzung sowohl die Anzucht (Kultivierung) als auch die Induktion der Mikroorganismen mit Nikotinsäure durchgeführt.

Vorzugsweise erfolgt die Kultivierung (Anzucht) und die Induktion mit Nikotinsäure als einzige Kohlenstoff-, Stickstoff und Energiequelle.

Vor der Substratzugabe (Pyrazincarbonsäure) können dann die Mikroorganismen entweder mittels üblichen Trennverfahren geerntet und in frischem Medium resuspendiert werden,oder das Substrat (Pyrazincarbonsäure) kann direkt zu den Mikroorganismen im ursprünglichen Wachstumsmedium zugegeben werden. Für das eigentliche Verfahren wird dann zweckmässig die Zellsuspension auf eine optische Dichte bei 650 nm, von 1 bis 100, vorzugsweise von 10 bis 30 eingestellt.

Als Medien können sowohl für die Kultivierung als auch für die eigentliche Umsetzung die in der Fachwelt üblichen angewendet werden. Vorzugsweise wird das Medium, dessen Zusammensetzung in Tabelle 1 angegeben ist, angewendet.

Das Substrat (Pyrazincarbonsäure) kann einmalig oder kontinuierlich zugegeben werden.

Zweckmässig erfolgt die Substratzugabe so, dass die Substratkonzentration 20 Gew%, vorzugsweise 5 Gew.%, nicht übersteigt. Üblicherweise erfolgt die Umsetzung der Pyrazincarbonsäure und/oder deren Salze zu 5-Hydroxypyrazincarbonsäure und/oder deren Salze mit ruhenden Zellen.

Zweckmässig wird die Umsetzung unter aeroben Bedingungen bei einem pH-Wert von 4 bis 10, vorzugsweise bei einem pH-Wert von 6 bis 8 durchgeführt.

Die Temperatur liegt zweckmässig zwischen 10 und 60°C, vorzugsweise zwischen 15 und 45°C.

Nach einer üblichen Umsetzungsdauer von 4 bis 100 h, kann das Produkt durch Ansäuern der zellfreien Lösung ausgefällt und mittels fachmännisch üblichen Aufarbeitungsmethoden erhalten werden. Im Falle des Natriumsalzes der 5-Hydroxypyrazincarbonsäure fällt das Produkt bereits während der Umsetzung aus.

### Beispiel 1:

Pseudomonas acidovorans DSM 4746 wurde in einem Mineralsalzmedium (Tabelle 1) unter einem kontinuierlichen Zusatz von Natriumnikotinat (0,6 g/l/h) in einem Fermenter bei pH 7,0 und bei einer Temperatur von 30°C bis zu einer optischen Dichte bei 650 nm von 10 angezogen.

Anschliessend wurden die Zellen abzentrifugiert und in 2 l einer Lösung, enthaltend 1 mol (146 g) Pyrazincarbonsäure-Natriumsalz, pH 7,0, resuspendiert.

Die optische Dichte bei 650 nm betrug dann 20.

Nach einer Inkubationszeit von 16 h unter aeroben Bedingungen bei pH 7,0 und einer Temperatur von 30°C konnte mittels UV-Spektroskopie kein Edukt mehr nachgewiesen werden. Ein Teil des gebildeten 5-Hydroxypyrazincarbonsäure-Natriumsalzes kristallisiertebereits unter diesen Versuchsbedingungen aus. Das ausgefallene Produkt wurde zusammen mit der Biomasse abzentrifugiert.

Das gebildete Sediment wurde anschliessend in 500 ml Wasser resuspendiert und erneut abzentrifugiert.

Die zellfreien Überstände wurden vereinigt und mit einem Rotationsverdampfer bis auf 300 ml eingeengt und auf 0°C abgekühlt. Die gebildeten Kristalle wurden abfiltriert und getrocknet. Insgesamt konnten 0,67 mol (108 g) 5-Hydroxypyrazincarbonsäure-Natriumsalz isoliert werden, entsprechend einer Ausbeute von 67%, bezogen auf eingesetztesPyrazincarbonsäure-Natriumsalz.

### Beispiel 2:

Pseudomonas acidovorans DSM 4746 wurde in einem Mineralsalzmedium (Tabelle 1) unter einem kontinuierlichen Zusatz von Natriumnikotinat (0,6 g/l/h) in einem Fermenter bei pH 7,0 und bei einer Temperatur von 30°C bis zu einer optischen Dichte bei 650 nm von 10 angezogen.

Anschliessend wurden die Zellen abzentrifugiert und in 100 ml einer Lösung, enthaltend 0,056 mol (7,9 g) Pyrazincarbonsäure-Ammoniumsalz, pH 7,0, resuspendiert.

Die optische Dichte bei 650 nm betrug dann 20.

Nach einer Inkubationszeit von 24 h unter aeroben Bedingungen bei pH 7,0 und einer Temperatur von 30°C konnte mittels UV-Spektroskopie kein Edukt mehr nachgewiesen werden. Danach wurde der zellfreie Überstand mit konzentrierter Schwefelsäure bis auf einen pH von 2,0 angesäuert um 5-Hydroxypyrazincarbonsäure auszufällen. Anschliessend wurde die Suspension auf 4°C gekühlt und filtriert.

Der Filtrationsrückstand wurde 2 Mal mit 10 ml Wasser gewaschen und getrocknet.

Insgesamt konnten 0,054 mol (7,56 g) 5-Hydroxypyrazincarbonsäure isoliert werden, entsprechend einer Ausbeute von 96%, bezogen auf eingesetztes Pyrazincarbonsäure-Ammoniumsalz.

Der Gehalt an 5-Hydroxypyrazincarbonsäure betrug mittels HPLC > 90%.

**Tabelle 1:**

| Zusammensetzung des Mineralsalzmediums | |
|---|---|
| | |
| - MgCl₂˙6H₂O | 0,8 g/l |
| - CaCl₂ | 0,16 g/l |
| - Na₂SO₄ | 0,25 g/l |
| - KH₂PO₄ | 0,4 g/l |
| - Na₂HPO₄ | 0,9 g/l |
| - SLF | 1 ml/l |
| - FeEDTA | 15 ml/l |
| | |

| Zusammensetzung der Spurenelemente (SLF) im Mineralsalzmedium | |
|---|---|
| | |
| - KOH | 15 g/l |
| - EDTANa₂˙2H₂O | 100 g/l |
| - ZnSO₄˙7H₂O | 9 g/l |
| - MnCl₂˙4H₂O | 4 g/l |
| - H₃BO₃ | 2,7 g/l |
| - CoCl₂˙6H₂O | 1,8 g/l |
| - CuCl₂˙2H₂O | 1,5 g/l |
| - NiCl₂˙6H₂O | 0,18 g/l |
| - Na₂MoO₄˙2H₂O | 0,2 g/l |
| | |

| Zusammensetzung von FeEDTA : | |
|---|---|
| | |
| - EDTA Na₂˙2H₂O | 5 g/l |
| - FeSO₄˙7H₂O | 2 g/l |
| | |
| (Der pH der Lösung wurde auf 7,0 eingestellt) | |

## Patentansprüche

1. Mikrobiologisches Verfahren zur Herstellung von 5-Hydroxypyrazincarbonsäure und/oder deren Salze, dadurch gekennzeichnet, dass man Pyrazincarbonsäure und/oder deren Salze, als Substrat, mit Mikroorganismen, die mit Nikotinsäure und/oder deren Salze als einzige Kohlenstoff-, Stickstoff- und Energieguelle wachsen und diese über 6-Hydroxynikotinsäure abbauen, in 5-Hydroxypyrazincarbonsäure und/oder deren Salze überführt, wobei letzteres im Medium akkumuliert wird.

2. Verfahren nach Patentanspruch 1, dadurch gekennzeichnet, dass man die Umsetzung mit Mikroorganismen der Gattungen Pseudomonas und/oder Achromobacter und/oder Bacillus und/oder Azorhizobium und/oder Sarcina und/oder Mycobacterium durchführt.

3. Verfahren nach mindestens einem der Patentansprüche 1 und 2, dadurch gekennzeichnet, dass man die Umsetzung mit Mikroorganismen der Spezies Pseudomonas acidovorans DSM 4746 oder deren Deszendenten und Mutanten durchführt.

4. Verfahren nach mindestens einem der Patentansprüche 1 und 2, dadurch gekennzeichnet, dass man die Umsetzung mit Mikroorganismen der Spezies Pseudomonas putida NCIB 10521 und/oder mit Mikroorganismen der Spezies Pseudomonas putida NCIB 8176 oder deren Deszendenten und Mutanten durchführt.

5. Verfahren nach mindestens einem der Patentansprüche 1 und 2, dadurch gekennzeichnet, dass man die Umsetzung mit Mikroorganismen der Spezies Achromobacter xylosoxydans DSM 2402 und/oder mit Mikroorganismen der Spezies Achromobacter xylosoxydans DSM 2783 oder deren Deszendenten und Mutanten durchführt.

6. Verfahren nach mindestens einem der Patentansprüche 1 bis 5, dadurch gekennzeichnet, dass die Umsetzung unter einmaliger oder kontinuierlicher Substratzugabe erfolgt, so dass die Substratkonzentration 20 Gew.% nicht übersteigt.

7. Verfahren nach mindestens einem der Patenansprüche 1 bis 6, dadurch gekennzeichnet, dass man die Umsetzung unter aeroben Bedingungen bei'einem pH von 4 bis 10 und bei einer Temperatur von 10 bis 60°C durchführt.

## Claims

1. A microbiological process for the preparation of 5-hydroxypyrazinecarboxylic acid and/or salts thereof, characterized in that the substrate pyrazinecarboxylic acid and/or salts thereof is converted to 5-hydroxypyrazinecarboxylic acid and/or salts thereof with microorganisms which grow with nicotinic acid and/or salts thereof as the only carbon, nitrogen and energy source and degrade the latter via 6-hydroxynicotinic acid, said 5-hydroxypyrazinecarboxylic acid and/or salts thereof accumulating in the medium.

2. A process according to Claim 1, characterized in that the reaction is carried out with microorganisms of the genera Pseudomonas and/or Achromobacter and/or Bacillus and/or Azorhizobium and/or Sarcina and/or Mycobacterium.

3. A process according to at least one of Claims 1 and 2, characterized in that the reaction is carried out with microorganisms of the species Pseudomonas acidovorans DSM 4746 or descendants and mutants thereof.

4. A process according to at least one of Claims 1 and 2, characterized in that the reaction is carried out with microorganisms of the species Pseudomonas putida NCIB 10521 and/or with microorganisms of the species Pseudomonas putida NCIB 8176 or descendants and mutants thereof.

5. A process according to at least one of Claims 1 and 2, characterized in that the reaction is carried out with microorganisms of the species Achromobacter xylosoxydans DSM 2402 and/or with microorganisms of the species Achromobacter xylosoxydans DSM 2783 or descendants and mutants thereof.

6. A process according to at least one of Claims 1 to 5, characterized in that the reaction is carried out with the substrate being added all at once or continuously so that the substrate concentration does not exceed 20 wt.%.

7. A process according to at least one of Claims 1 to 6, characterized in that the reaction is carried out under aerobic conditions at a pH of 4 to 10 and at a temperature of 10 to 60°C.

## Revendications

1. Procédé microbiologique pour la préparation de l'acide 5-nydroxypyrazinecarboxylique et/ou de ses sels, caractérisé en ce que l'on transforme l'acide pyrazinecarboxylique et/ou ses sels, comme substrat, avec des microorganismes, qui sont en culture avec l'acide nicotinique et/ou ses sels comme seule et unique source de carbone, d'azote et d'énergie et dégrade ceux-ci par l'intermédiaire de l'acide 6-hydroxynicotinique, on transforme en l'acide 5-hydroxypyrazinecarboxylique et/ou en ses sels, ces derniers étant accumulés dans le milieu.

2. Procédé selon la revendication 1, caractérisé en ce que l'on conduit la réaction avec des microorganismes des espèces Pseudomonas et/ou Achromobacter et/ou Bacillus et/ou Azorhizobium et/ou Sarcina et/ou Mycobacterium.

3. Procédé selon au moins l'une des revendications 1 et 2, caractérisé en ce que l'on conduit la réaction avec des microorganismes de l'espèce Pseudomonas acidovorans DSM 4746 ou de leurs descendants et mutants.

4. Procédé selon au moins l'une des revendications 1 et 2, caractérisé en ce que l'on conduit la réaction avec des microorganismes de l'espèce Pseudomonas putida CNIB 10521 et/ou avec des microorganismes de l'espèce Pseudomonas putida NCIB 8176 ou de leurs descendants et mutants.

5. Procédé selon au moins l'une des revendications 1 et 2, caractérisé en ce que l'on conduit la réaction avec des microorganismes de l'espèce Achromobacter xylosoxydans DSM 2402 et/ou avec des microorganismes de l'espèce Achromobacter xylosoxydans DSM 2783 ou de leurs descendants et mutants.

6. Procédé selon au moins l'une des revendications 1 à 5, caractérisé en ce que la réaction s'effectue par addition de substrat en une seule fois ou de façon continue afin que la concentration de substrat ne dépasse pas 20 % en poids.

7. Procédé selon au moins l'une des revendications 1 à 6, caractérisé en ce que l'on conduit la réaction dans des conditions aérobies avec un pH de 4 à 10 et une température de 10 à 60°C.
